# EUROPÄISCHE PATENTSCHRIFT

(11) **EP 0 934 246 B1**
(45) Veröffentlichungstag und Bekanntmachung des Hinweises auf die Patenterteilung: **16.05.2001**
(21) Anmeldenummer: 97909373.9
(22) Anmeldetag: 09.10.1997
(51) Int. Cl.: C07C 67/31, C07C 69/734, C07C 51/09, C07C 59/64, C07C 67/343

(54) **VERFAHREN ZUR HERSTELLUNG VON ALPHA-ALKOXY-ALPHA-TRIFLUORMETHYL-ARYLESSIGSÄUREESTERN UND -ARYLESSIGSÄUREN**
METHOD FOR PRODUCING ALPHA-ALKOXY-ALPHA-TRIFLUOROMETHYL-ARYL ACETIC ACID ESTERS AND -ARYL ACETIC ACIDS
PROCEDE DE PRODUCTION D'ESTERS ET D'ACIDES ALPHA-ALCOXY-ALPHA-TRIFLUOROMETHYL-ARYL ACETIQUES

(30) Priorität: 22.10.1996 DE 19643592
(43) Veröffentlichungstag der Anmeldung: 11.08.1999
(73) Patentinhaber: BAYER AG, 51368 Leverkusen (DE)
(72) Erfinder: LUI, Norbert, D-51061 Köln (DE); MARHOLD, Albrecht, D-51373 Leverkusen (DE)
(86) Internationale Anmeldenummer: EP9705565
(87) Internationale Veröffentlichungsnummer: WO9817622

(56) Entgegenhaltungen:
- PICHIKA RAMAIAH ET AL.: "Direct Trifluoromethylation of alpha-Keto Esters to beta,beta,beta-Trifluorolactic Acid Derivatives Using Trifluoromethyltrimethylsilane" SYNLETT., Nr. 9, September 1991, STUTTGART DE, Seiten 643-644, XP002052911 in der Anmeldung erwähnt

## Beschreibung

Die vorliegende Erfindung betrifft ein vorteilhaftes Verfahren zur Herstellung von α-Alkoxy-α-trifluormethyl-arylessigsäureestern und -arylessigsäuren aus Arylketoestern. α-Alkoxy-α-trifluormethyl-arylessigsäuren, z.B. α-Methoxy-α-trifluor-methyl-phenylessigsäure, die sog. Mosher-Säure, sind wichtige Reagenzien zur Bestimmung der optischen Reinheit von chiralen Aminen (siehe Synlett 1991, 643).

Verfahren zur Herstellung von α-Methoxy-α-trifluormethyl-phenylessigsäure sind bekannt, jedoch sind die bekannten Verfahren alle unbefriedigend.

So wird gemäß J. Org. Chem. 34, 2543 (1969) Natriumcyanid mit α,α,α-Trifluor-acetophenon in 1,2-Dimethoxyethan umgesetzt, anschließend mit Dimethylsulfat alkyliert, dann das Nitril zum Amid und abschließend das Amid zur Säure verseift. Bei einer Variation dieses Verfahrens wird anstelle des Methoxyethans t.-Butanol verwendet und die Verseifungen mit alkalischer Wasserstoffperoxidlösung durchgeführt (Tetrahedron 42, 547 (1986)). Nachteilig ist in beiden Fällen die schlechte Zugänglichkeit des Trifluoracetophenons, bei dessen Herstellung gasförmiges Trifluoracetylchlorid (Siedepunkt: +2°C) gehandhabt werden muß, und daß Trifluoracetophenon nur mit mäßigen Ausbeuten erhältlich ist. Ferner muß toxisches Natriumcyanid gehandhabt werden, was besonderen Aufwand, auch für die Entsorgung bedeutet. Schließlich tritt bei den Verseifungen als Nebenkomponente Benzoesäure auf, die wegen ihrer Sublimationsfähigkeit nur schwer abzutrennen ist, weshalb auf diese Weise im allgemeinen nur Benzoesäure enthaltende Produkte erhalten werden können.

Ein anderes Verfahren zur Herstellung von a-Methoxy-a-trifluormethyl-phenyl-essigsäure (J. Org. Chem. 57, 3731 (1992)) geht von Trifluoressigsäuretrimethyl-silylestern und α,α,α-Trifluoracetophenon aus, die in Gegenwart des Kronenethers 18-Krone-6 umgesetzt werden. Anschließend wird die Trichlormethylgruppe mit Kaliumhydroxid/Methanol verseift, wobei wiederum Benzoesäure als Nebenkomponente anfällt. Neben den oben beschriebenen Nachteilen, die durch den Einsatz von α,α,α-Trifluoracetophenon und die als Nebenkomponente entstehende Benzoesäure verursacht werden, ist hier außerdem ein teurer Kronenether zu verwenden, der auch Entsorgungsprobleme verursacht.

Schließlich beschreibt Synlett (loc.cit.) die Umsetzung von α-Ketoestern mit Trifluormethyl-trimethylsilan und die Verseifung des gebildeten Trifluormethyl-trimethylsilylethers mit wäßriger Salzsäure zur entsprechenden Trifluormethyl-hydroxyverbindung. Werden Benzylester eingesetzt, so wird bis zur Hydroxyverbindung eine Reaktionszeit von 69 Stunden benötigt (loc.cit., Tabelle 1, 4. Zeile), das sind fast 3 volle Tage. Im Falle von Benzoylformiaten (= Benzoyl-alkoxyverbindungen) wird angegeben, daß die Hydrolyse der Silylester Schwierigkeiten bereitet. Eine brauchbare Synthese von α-Alkoxy-α-trifluormethyl-arylessigsäuren ist so also nicht durchführbar.

Es wurde nun ein Verfahren zur Herstellung von a-Alkoxy-α-trifluormethylarylessigsäureestern der Formel (I) gefunden 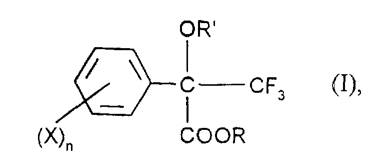 in der
- R: für C₁-C₆-Alkyl, C₂-C₆-Alkenyl, C₃-C₇-Cycloalkyl, C₆-C₁₀-Aryl oder C₁-C₆-Halogenalkyl,
- R': für C₁-C₄-Alkyl,
- X: für gleiche oder verschiedene Substituenten aus der Gruppe Halogen, C₁-C₄-Alkyl, C₂-C₄-Alkenyl, C₆-C₁₀-Aryl oder C₁-C₄-Alkoxy und Nitro
und
- n: für Null oder eine ganze Zahl von 1 bis 3 stehen,
das dadurch gekennzeichnet ist, daß man einen Ketoester der Formel 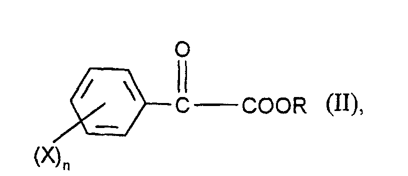 in der
R, X und n die bei Formel (I) angegebene Bedeutung haben,
in Gegenwart eines Lösungsmittels und eines im Reaktionsgemisch zumindest geringfügig löslichen Fluorids als Katalysator, mit Trifluormethyl-trimethylsilan umsetzt, so einen Trimethylsilylether der Formel (III) erhält 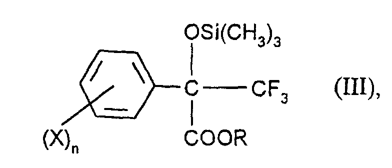 in der
R, X und n die bei Formel (I) angegebene Bedeutung haben,
diesen mit einem Alkoholat oder einem alkalisch eingestellten Alkohol umsetzt, so das Salz einer Hydroxyverbindung der Formel (IV) erhält, 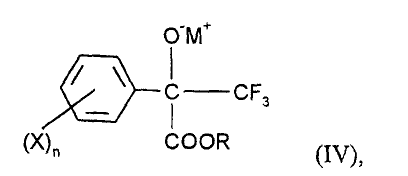 in der
R, X und n die bei Formel (I) angegebene Bedeutung haben und M⁺ für das Ion eines Alkalimetalls steht,
und dieses mit einem Alkylierungsmittel umsetzt, das die O⁻M⁺-Gruppe in eine OR'-Gruppe überführt.

Soweit R für C₁-C₆-Halogenalkyl steht kann es sich z.B. um Alkylgruppen handeln, die geradkettig oder verzweigt sind und die 1 bis 5 gleiche oder verschiedene Substituenten aus der Gruppe Fluor, Chlor und Brom enthalten.

Vorzugsweise steht R für geradkettiges oder verzweigtes C₁-C₄-Alkyl, C₂-C₄-Alkenyl, Cyclopentyl, Cyclohexyl, Phenyl oder C₁-C₄-Halogenalkyl, das 1 bis 5 Fluor- und/oder Chloratome enthält. Besonders bevorzugt steht R für Methyl oder Ethyl.

R' kann geradkettig oder verzweigt sein und beispielsweise für Methyl, Ethyl oder Isopropyl stehen. Vorzugsweise steht es für Methyl.

X steht vorzugsweise für gleiche oder verschiedene Substituenten aus der Gruppe Methyl, Chlor, Brom, Ethenyl, Phenyl und Nitro.

n steht vorzugsweise für Null, 1 oder 2, insbesondere für Null.

In das erfindungsgemäße Verfahren einsetzbare Ketoester der Formel (II) sind z.B. im Handel erhältlich oder auf bekannte Weise oder analog dazu herstellbar.

Trifluormethyl-trimethylsilan ist im Handel erhältlich.

Das Molverhältnis des Ketoesters der Formel (II) zu Trifluormethyl-trimethylsilan kann z.B. 1:1 bis 1:2 betragen. Vorzugsweise liegt es bei 1:1 bis 1:1,5.

Als Lösungsmittel für diese Umsetzung kommen z.B. aprotische Lösungsmittel infrage, wie Ether, Alkylchloride, Amide (auch cyclische), Nitrile und aromatische Kohlenwasserstoffe. Bevorzugt sind Tetrahydrofuran, Polyether der Formel CH₃O-(CH₂-CH₂-O)ₘ-CH₃ mit m = 1 bis 4, Methylenchlorid, Chloroform, Dimethylformamid, N-Methylpyrrolidon, Acetonitril und Toluol.

Die Umsetzung wird vorzugsweise mit weitgehend wasserfreien Lösungsmitteln und Reaktanden durchgeführt.

Als im Reaktionsgemisch zumindest geringfügig lösliche, als Katalysator geeignete Fluoride sind z.B. Alkalifluoride, Alkalibifluoride, quartäre Oniumfluoride, chirale Fluoride und Tris-(dimethylamino)-sulfoniumdifluortrimethylsilikat anwendbar. Als weitere Einzelverbindungen seien genannt: Kaliumfluorid, Cäsiumfluorid, Kaliumhydrogenbifluorid, Tetrabutylammoniumfluorid, -hydrogenbifluorid, -dihydrogentrifluorid, Tetramethyl- und Tetraethylammoniumfluoride, Tetraalkyl- und Tetraphenylphosphoniumfluoride und N-Benzylcinchoniumfluorid. Der Fluoridkatalysator kann, soweit es sich um organische Fluoride handelt, gegebenenfalls Hydratwasser enthalten.

Gegebenenfalls kann man zusätzlich Kronenether einsetzen, z.B. 18-Krone-6 oder dessen Addukt mit Kaliumfluorid. Die Menge der Kronenether kann beispielsweise von 1 bis 20 Mol-%, bezogen auf den Fluoridkatalysator, betragen. Beim Einsatz von organischen Gruppen enthaltenden Fluoriden bringt die Verwendung von Kronenethern im allgemeinen keinen Vorteil.

Bevorzugt setzt man Kaliumfluorid, Cäsiumfluorid oder Tetrabutylammoniumfluorid, -hydrogenfluorid oder -dihydrogentrifluorid ein.

Sofern der Fluoridkatalysator in einem für die Umsetzung geeigneten Lösungsmittel löslich ist, kann man ihn auch in gelöster Form einsetzen, z.B. eine Lösung von Tetrabutylammoniumfluorid x 3H₂O in Tetrahydrofuran.

Die Menge des Fluoridkatalysators kann z.B. 0,05 bis 30 Mol-%, vorzugsweise 0,1 bis 20 Mol-%, bezogen auf den Ketoester der Formel (II), betragen.

Die Umsetzung des Ketoesters der Formel (II) mit Trifluormethyl-trimethylsilan kann z.B. bei -40 bis 120°C, bevorzugt bei 0 bis 80°C durchgeführt werden. Der Druck ist nicht kritisch. Es kann bei Normaldruck, erhöhtem Druck oder erniedrigten Druck gearbeitet werden. Vorzugsweise arbeitet man bei Normaldruck oder im geschlossenen Gefäß bei dem sich unter Reaktionstemperatur von selbst entstellenden Druck.

Die Umsetzung des Ketoesters der Formel (II) mit Trifluormethl-trimethylsilan kann man z.B. so durchführen, daß man das Lösungsmittel, den Ketoester und den Katalysator vorlegt und das Silan zudosiert oder, daß man das Lösungsmittel, den Ketoester und das Silan vorlegt und den Katalysator zudosiert. Auch andere Durchführungsmöglichkeiten dieser Umsetzung sind möglich.

Nach Beendigung der Reaktion kann gewünschtenfalls das Lösungsmittel entfernt werden, z.B. durch Destillation. Es ist jedoch bevorzugt, den erhaltenen Trimethylsilylether der Formel (III) in Form des ausreagierten Reaktionsgemisches mit einem Alkoholat oder einem alkalisch eingestellten Alkohol umzusetzen.

Als Alkoholate kommen z.B. aliphatische C₁-C₄-Alkoholate von Alkalimetallen infrage, wie Natriummethylat, Natriumethylat und Kalium-t-butylat. Vorzugsweise setzt man die Alkoholate gelöst in dem entsprechenden Alkohol ein, also z.B. Natriummethylat gelöst in Methanol.

Als alkalisch eingestellte Alkohole kommen z.B. aliphatische C₁-C₄-Alkohole infrage, die mit Alkalihydroxiden und/oder Alkalicarbonaten alkalisch eingestellt wurden. Als Beispiele seien genannt: Mischungen aus Natriumhydroxid und Methanol sowie Mischungen aus Kaliumcarbonat und Ethanol.

Die Alkoholate und die alkalisch eingestellten Alkohole werden vorzugsweise in möglichst wasserfreier Form eingesetzt. Vorzugsweise wird ein Alkalialkoholat eingesetzt, bei dem der Alkoholatrest identisch ist mit der OR'-Gruppe im eingesetzten Ketoester der Formel (II).

Die Mengen an Alkoholaten bzw. alkalisch eingestellten Alkoholen können z.B. so gewählt werden, daß, bezogen auf auf den Trimethylsilylether der Formel (III), 1 bis 5 Äquivalent-% Alkoholat bzw. % alkalische Äquivalente im alkalisch eingestellten Alkohol zum Einsatz gelangen. Vorzugsweise beträgt diese Menge von 1 bis 1,5 Äquivalent-% bzw. % alkalische Äquivalente

Sofern zusammen mit dem Alkoholat oder in Form des alkalisch eingestellten Alkohols nicht genügend Alkohol in das Reaktionsgemisch eingebracht oder aus der vorhergehenden Reaktionsstufe nicht genügend Lösungsmittel vorhanden ist, ist es vorteilhaft, weiteren Alkohol als Lösungsmittel einzusetzen, um ein gut handhabbares Reaktionsgemisch zu erhalten. Vorzugsweise nimmt man einen Alkohol, dessen OR'-Gruppe derjenigen des eingesetzten Ketoesters der Formel (II) entspricht.

Die Umsetzung mit dem Alkoholat bzw. dem alkalisch eingestellten Alkohol kann z.B. bei Temperaturen im Bereich -20 bis +120°C, vorzugsweise im Bereich 0 bis 80°C durchgeführt werden. Der Druck ist auch hier nicht kritisch. Es kann bei Normaldruck, erhöhtem Druck oder erniedrigtem Druck gearbeitet werden. Vorzugsweise arbeitet man Normaldruck oder im geschlossenen Gefäß bei dem sich unter Reaktionstemperatur von selbst einstellenden Druck.

Man kann die Umsetzung mit dem Alkoholat bzw. dem alkalisch eingestellten Alkohol vorteilhafter Weise so durchführen, daß man das Alkoholat als Lösung in einem Alkohol oder den alkalisch eingestellten Alkohol zu dem ausreagierten Reaktionsgemisch der vorherigen Reaktionsstufe zudosiert. Auch andere Durchführungsformen dieser Reaktion sind möglich.

Nach Beendigung der Reaktion ist es bevorzugt, vorhandene Lösungsmittel zu entfernen, z.B. durch Abziehen im Vakuum. Man kann aber auch das gesamte Reaktionsgemisch zur weiteren Umsetzung verwenden.

Zur Umsetzung des erhaltenen Salzes einer Hydroxyverbindung der Formel (IV) mit einem Alkylierungsmittel kann man z.B. an sich bekannte Alkylierungsmittel einsetzen, beispielsweise Methylierungsmittel wie Dimethylsulfat, Methyliodid, Methylbromid, Methylchlorid oder Toluolsulfonsäuremethylestern oder Ethylierungsmittel wie Ethyliodid oder Toluolsulfonsäureethylester. Bezogen auf die eingesetzte Hydroxyverbindung der Formel (IV) kann man z.B. 1 bis 5 Äquivalente eines Alkylierungsmittels verwenden. Vorzugsweise liegt diese Menge bei 1 bis 2 Äquivalenten.

Die Temperatur kann bei der Umsetzung beispielsweise zwischen 0 und 100°C betragen, vorzugsweise liegt sie bei 5 bis 80°C. Wenn man bei Reaktionstemperatur gasförmige Alkylierungsmittel einsetzt, ist es erforderlich unter Druck zu arbeiten, beispielsweise bei bis zu 10 bar. Sonst ist der Druck nicht kritisch. Es kann dann bei Normaldruck, erniedrigtem Druck oder erhöhtem Druck gearbeitet werden. Besonders bevorzugt verwendet man als Alkylierungsmittel Dimethylsulfat und arbeitet bei Normaldruck.

Bei der Alkylierung ist der Zusatz eines Lösungsmittels nicht unbedingt erforderlich, insbesondere wenn aus der vorherigen Reaktionsstufe schon eines mit gebracht wurde. Gewünschtenfalls kann man ein inertes Lösungsmittel zufügen, z.B. einen Ether wie Methyl-tert.-butylether oder einen Kohlenwasserstoff wie Toluol oder Hexan.

Die Umsetzung kann man z.B. so durchführen, daß man das Salz der Hydroxyverbindung der Formel (IV), gegebenenfalls in Form des bei seiner Herstellung anfallenden ausreagierten Reaktionsgemisches und gegebenenfalls Lösungsmittel vorlegt und das Alkylierungsmittel zudosiert. Es sind auch andere Formen der Durchführung dieser Reaktion möglich. Den Umsatz des Salzes der Hydroxyverbindung der Formel (IV) kann man gegebenenfalls vervollständigen, indem man Base nachdosiert. Geeignet hierfür sind beispielsweise die weiter oben beschriebenen Alkoholate und wäßrige Lösungen von Alkalihydroxiden.

Die Aufarbeitung des Reaktionsgemisches und die Isolierung des hergestellten α-Alkoxy-α-trifluormethyl-arylessigsäureesters der Formel (I) kann durch Destillation erfolgen. Man kann das Reaktionsgemisch auch auf Wasser austragen, falls nötig mit einer Säure neutralisieren (z.B. bis zu einem pH-Wert im Bereich von 6 bis 8), die Verbindung der Formel (I) mit einem geeigneten Extraktionsmittel, z.B. Methyl-tert.-methylether, extrahieren und durch Abziehen des Extraktionsmittels isolieren. Das isolierte Produkt der Formel (I) kann auf einfache Weise weiter gereinigt werden, z.B. durch Destillation. Hierbei können, im Gegensatz zum Stand der Technik, keine Probleme mit als Nebenkomponente entstandender Benzoesäure auftreten. Beim erfindungsgemäßen Verfahren können allenfalls Benzoesäureester als Nebenkomponente auftreten und diese können, im Gegensatz zur Benzoesäure, durch Destillation einfach abgetrennt werden, da sie keine Tendenz zur Sublimation aufweisen.

Erfindungsgemäß können auch α-Alkoxy-α-trifluormethyl-arylessigsäuren der Formel (V) hergestellt werden 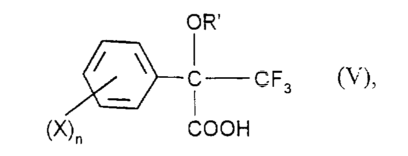 in der R', X und n die bei Formel (I) angegebene Bedeutung haben. Hierzu stellt man zunächst wie oben beschrieben, α-Alkoxy-α-trifluormethyl-arylessigsäureester der Formel (I) her, und führt diese durch Hydrolyse oder Spaltung mit einer Säure in eine Säure der Formel (V) über.

Für die Hydrolyse können alkalische oder saure wäßrige Lösungen verwendet werden, z.B. wäßrige Lösungen von Alkalihydroxiden oder Mineralsäuren. Man kann dabei nach üblichen Methoden der Esterverseifung arbeiten.

Gegebenenfalls kann man in Gegenwart eines Verdünnungsmittels arbeiten, z.B. eines Alkohols wie Methanol.

Die Spaltung mit einer Säure kann z.B. mit Ameisensäure nach der in Org. Synth. Coll. Vol. III, 33 (1955) beschriebenen Methode vorgenommen werden

Bevorzugt wird auf die erfindungsgemäße Weise die sog. Mosher-Säure hergestellt (Formel (V); R' = CH₃, n = Null).

Das erfindungsgemäße Verfahren gestattet die Herstellung von α-Alkoxy-α-trifluormethyl-arylessigsäureestern und -arylessigsäuren auf sehr günstige Weise. So sind die Reaktionszeiten kurz, die benötigten Reagenzien gut zugänglich, der Umgang mit toxischen Natriumcyanid ist nicht erforderlich, die Nebenkomponente Benzoesäure, die nur schwierig abtrennbar ist, kann nicht entstehen, und die Ausbeuten und Reinheiten der Produkte sind stark verbessert. Die Ester der Formel (I) sind aus Keotestern der Formel (II) beispielsweise in Ausbeuten von weit über 85 % der Theorie und die Säuren der Formel (V) aus den Estern der Formel (I) beispielsweise in Ausbeuten von weit über 90 % der Theorie zugänglich. Hinzu kommt, daß man die Ester der Formel (I) ohne Zwischenisolierung der Verbindungen der Formeln (III) und (IV) in einem Eintopfverfahren herstellen kann, was einen nur geringen apparativen Aufwand bedeutet.

Im Hinblick auf den eingangs geschilderten Stand der Technik sind die mit den erfindungsgemäßen Verfahren erzielbaren positiven Effekte außerordentlich überraschend.

### Beispiele

### Beispiel 1

164 g Phenylgloxylsäuremethylester (Formel II); R = CH₃, n = Null) und 156 g Trifluormethyl-trimethylsilan gelöst in 400 ml Tetrahydrofuran wurden bei Raumtemperatur unter Stickstoff vorgelegt und anschließend eine Lösung von 1 g Tetrabutylammoniumfluorid x 3H₂O in 50 ml Tetrahydrofuran zugetropft. Nach 3 Stunden wurden in das dann vorliegende Reaktionsgemisch 216 g 30 gew.-%ige Natriummethylatlösung in Methanol zugetropft und 1 Stunde bei Raumtemperatur nachgerührt. Es fiel ein farbloser Feststoff aus. Die vorhandenen Lösungsmittel wurden im Vakuum entfernt und dem zurückbleibenden Feststoff anschließend 500 ml Methyl-tert.-butylether zugesetzt und dann 170 g Dimethylsulfat bei 25°C zugetropft. Abschließend wurden 25 ml 20 gew.-%ige wäßrige Natriumhydroxidlösung zugesetzt. Nach 3 Stunden wurden 500 ml Wasser hinzugefügt, die organische Phase abgetrennt, die wäßrige Phase mit Methyl-tert.-butylether extrahiert, über Natriumsulfat getrocknet und destilliert. Es wurden 211 g α-Methoxy-α-trifluormethylphenylessigsäuremethylester (90 % d. Theorie) isoliert. (Kp: 62°C/0,03 mm).

### Beispiel 2

210 g α-Methoxy-α-trifluormethylphenylessigsäuremethylester (erhalten gemäß Beispiel 1) wurden in 600 ml Methanol bei 25°C vorgelegt und 860 ml IN wäßrige Natronlauge langsam zugetropft. Es wurde 5 Stunden bei Raumtemperatur gerührt. Anschließend wurde mit verdünnter wäßriger Salzsäure ein pH-Wert von 1 eingestellt, 2 x mit je 200 ml Methylenchlorid extrahiert, die organische Phase über Natriumsulfat getrocknet und abschließend das Lösungsmittel entfernt. So wurden in 96 %iger Ausbeute a-Methoxy-α-trifluormethylphenylessigsäure erhalten (= 190 g).

## Patentansprüche

1. Verfahren zur Herstellung von a-Alkoxy-a-trifluormethyl-arylessigsäureestern der Formel (I) 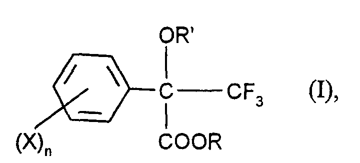 in der
R für C₁-C₆-Alkyl, C₂-C₆-Alkenyl, C₃-C₇-Cycloalkyl, C₆-C₁₀-Aryl oder C₁-C₆-Halogenalkyl,
R' für C₁-C₄-Alkyl,
X für gleiche oder verschiedene Substituenten aus der Gruppe Halogen, C₁-C₄-Alkyl, C₂-C₄-Alkenyl, C₆-C₁₀-Aryl oder C₁-C₄-Alkoxy und Nitro
und
n für Null oder eine ganze Zahl von 1 bis 3 stehen,
dadurch gekennzeichnet, daß man einen Ketoester der Formel (II) 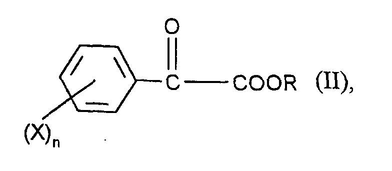 in der
R, X und n die bei Formel (I) angegebene Bedeutung haben,
in Gegenwart eines Lösungsmittels und eines im Reaktionsgemisch zumindest geringfügig löslichen Fluorids als Katalysator, mit Trifluormethyl-trimethylsilan umsetzt, so einen Trimethylsilylether der Formel (III) erhält 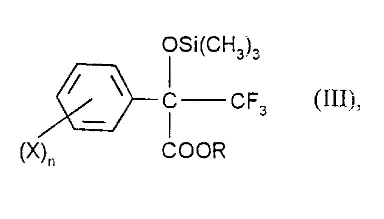 in der
R, X und n die bei Formel (I) angegebene Bedeutung haben,
diesen mit einem Alkoholat oder einem alkalisch eingestellten Alkohol umsetzt, so eine entsprechende Hydroxyverbindung der Formel (IV) erhält, 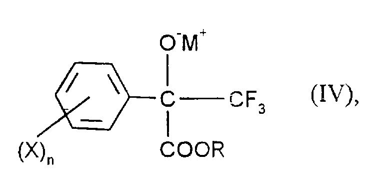 in der
R, X und n die bei Formel (I) angegebene Bedeutung haben und M⁻ für das Ion eines Alkalimetalls steht,
und diese mit einem Alkylierungsmittel umsetzt, das die OH-Gruppe in eine OR'-Gruppe überführt.

2. Verfahren nach Anspruch 1, dadurch gekennzeichnet, daß in den Formeln
R für geradkettiges oder verzweigtes C₁-C₄-Alkyl, C₂-C₄-Alkylen, Cyclopentyl, Cyclohexyl, Phenyl oder C₁-C₄-Halogenalkyl mit 1 bis 5 Fluor- oder Chloratomen,
R' für Methyl, Ethyl oder Isopropyl,
X für gleiche oder verschiedene Substituenten aus der Gruppe Methyl, Chlor, Brom, Ethenyl, Phenyl und Nitro,
n für Null, 1 oder 2 stehen.

3. Verfahren nach Ansprüchen 1 und 2, dadurch gekennzeichnet, daß das Molverhältnis der Ketoverbindung der Formel (II) zu Trifluormethyl-trimethylsilan 1:1 bis 1:2 beträgt, als Lösungsmittel für die Umsetzung der Ketoverbindung der Formel (II) mit Trifluormethyl-trimethylsilan ein aprotisches Lösungsmittel eingesetzt wird, als Fluoridkatalysator 0,05 bis 30 Mol-%, bezogen auf den Ketoester der Formel (II) Alkalifluoride, Alkalibifluoride, quartäre Oniumfluoride, chirale Fluoride oder Tris(dimethylamino)-sulfoniumdifluortrimethylsilikat verwendet wird und die Umsetzung des Ketoesters der Formel (II) mit Trifluormethyl-trimethylsilan bei -40 bis +120°C durchgeführt wird.

4. Verfahren nach Ansprüchen 1 bis 3, dadurch gekennzeichnet, daß man als Alkoholate aliphatische C₁-C₄-Alkoholate von Alkalimetallen oder als alkalisch eingestellte Alkohole aliphatische C₁-C₄-Alkohole einsetzt, die mit Alkalihydroxiden und/oder Alkalicarbonaten alkalisch eingestellt wurden, die Alkoholate bzw die alkalisch eingestellten Alkohole in einer Menge von 1 bis 5 Äquivalent-%, bezogen auf den Trimethylsilylether der Formel (III) einsetzt und die Umsetzung mit dem Alkoholat bzw. dem alkalisch eingestellten Alkohol bei 0 bis 80°C durchführt.

5. Verfahren nach Ansprüchen 1 bis 4, dadurch gekennzeichnet, daß man als Alkylierungsmittel an sich bekannte Methylierungsmittel oder Ethylierungsmittel in einer Menge von 1 bis 5 Äquivalenten, bezogen auf das Salz einer Hydroxyverbindung der Formel (IV) einsetzt und die Alkylierung bei Temperaturen zwischen und 0 und 100°C vornimmt.

6. Verfahren nach Ansprüchen 1 bis 5, dadurch gekennzeichnet, daß man zur Gewinnung des hergestellten α-Alkoxy-α-trifluormethyl-arylessigsäureesters der Formel (I), das nach der Alkylierung vorliegende Reaktionsgemisch durch Destillation oder durch Austragen auf Wasser, Neutralisation mit einer Säure, Extraktion mit einem geeigneten Lösungsmittel und Abziehen des Extraktionsmittels isoliert.

7. Verfahren nach Ansprüchen 1 bis 6, dadurch gekennzeichnet, daß man den aus dem Reaktionsgemisch der Alkylierung isolierten α-Alkoxy-α-trifluormethyl-arylessigsäureester der Formel (I) durch Destillation weiter reinigt.

8. Verfahren zur Herstellung von α-Alkoxy-α-trifluormethyl-arylessigsäuren der Formel (V) 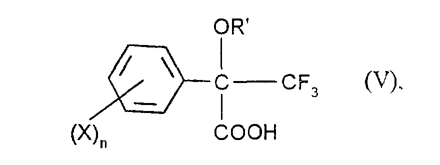 in der
R', X und n die bei Formel (I) in Anspruch 1 angegebene Bedeutung haben, dadurch gekennzeichnet, daß man zunächst nach den Ansprüchen 1 bis 8 α-Alkoxy-α-trifluormethyl-arylessigsäureester der Formel (I) herstellt, und diese durch Hydrolyse oder Spaltung mit einer Säure in eine α-Alkoxy-α-trifluormethyl-arylessigsäure der Formel (V) überführt.

## Claims

1. Process for the preparation of a-alkoxy-a-trifluoromethyl-arylacetic esters of the formula (I) 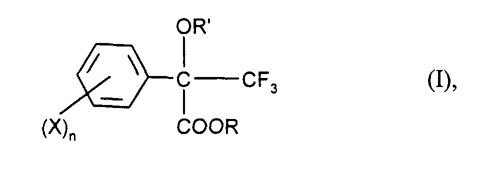 in which
R is C₁-C₆-alkyl, C₂-C₆-alkenyl, C₃-C₇-cycloalkyl, C₆-C₁₀-aryl or C₁-C₆-halogenoalkyl,
R' is C₁-C₄-alkyl,
X is identical or different substituents from the group consisting of halogen, C₁-C₄-alkyl, C₂-C₄-alkenyl, C₆-C₁₀-aryl or C₁-C₄-alkoxy and nitro
and
n is zero or an integer from 1 to 3,
characterized in that a keto ester of the formula (II) 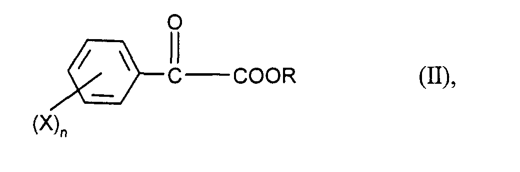 in which
R, X and n are as defined in formula (I),
is reacted with trifluoromethyl-trimethylsilane in the presence of a solvent and of a fluoride at least slightly soluble in the reaction mixture, as catalyst to give a trimethyl silyl ether of the formula (III) 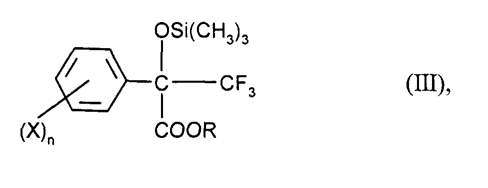 in which
R, X and n are as defined in formula (I),
which is reacted with an alkoxide, or an alcohol which has been rendered alkaline to give a corresponding hydroxyl compound of the formula (IV), 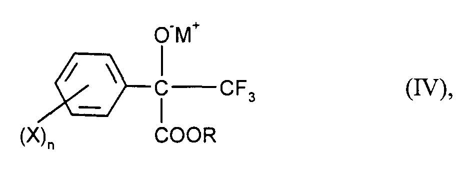 in which
R, X and n are as defined in formula (I), and M⁺ is the ion of an alkali metal,
which is reacted with an alkylating agent, which converts the OH group into an OR' group.

2. Process according to Claim 1, characterized in that, in the formulae
R is a straight-chain or branched C₁-C₄-alkyl, C₂-C₄-alkylene, cyclopentyl, cyclohexyl, phenyl or C₁-C₄-halogenoalkyl having from 1 to 5 fluorine or chlorine atoms,
R' is methyl, ethyl or isopropyl,
X is identical or different substituents from the group consisting of methyl, chlorine, bromine, ethenyl, phenyl and nitro,
n is zero, 1 or 2.

3. Process according to Claims 1 and 2, characterized in that the molar ratio of the keto compound of the formula (II) to trifluoromethyl-trimethylsilane is from 1:1 to 1:2, the solvent used for the reaction of the keto compound of the formula (II) with trifluoromethyl-trimethylsilane is an aprotic solvent, the fluoride catalyst used is from 0.05 to 30 mol%, based on the keto ester of the formula (II), of alkali fluorides, alkali metal bifluorides, quaternary onium fluorides, chiral fluorides or tris(dimethylamino)-sulphonium difluorotrimethylsilicate, and the reaction of the keto ester of the formula (II) with trifluoromethyl-trimethylsilane is carried out at from -40 to +120°C.

4. Process according to Claims 1 to 3, characterized in that the alkoxides used are aliphatic C₁-C₄-alkoxides of alkali metals, or the alcohols rendered alkaline used are aliphatic C₁-C₄-alcohols which have been rendered alkaline using alkali metal hydroxides and/or alkali metal carbonates, the alkoxides or the alcohols which have been rendered alkaline are used in an amount of from 1 to 5 equivalent%, based on the trimethyl silyl ether of the formula (III), and the reaction with the alkoxide or the alcohol which has been rendered alkaline is carried out at from 0 to 80°C.

5. Process according to Claims 1 to 4, characterized in that the alkylating agents used are methylating agents or ethylating agents known per se in an amount of from 1 to 5 equivalents, based on the salt of a hydroxyl compound of the formula (IV), and the alkylation is carried out at temperatures between 0 and 100°C.

6. Process according to Claims 1 to 5, characterized in that, in order to obtain the prepared α-alkoxy-α-trifluoromethyl-arylacetic ester of the formula (I), the reaction mixture produced after alkylation is isolated by distillation or by discharge into water, neutralization with an acid, extraction with a suitable solvent and removal of the extractant.

7. Process according to Claims 1 to 6, characterized in that the α-alkoxy-α-trifluoromethyl-arylacetic ester of the formula (I) isolated from the alkylation reaction mixture is further purified by distillation.

8. Process for the preparation of α-alkoxy-α-trifluoromethyl-arylacetic acids of the formula (V) 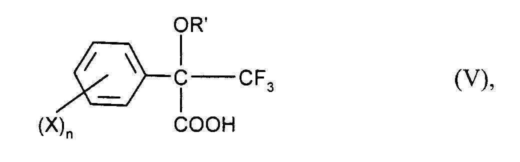 in which
R', X and n are as defined in formula (I) in Claim 1, characterized in that α-alkoxy-α-trifluoromethyl-arylacetic esters of the formula (I) are firstly prepared according to Claims 1 to 8, and these are converted into an α-alkoxy-α-trifluoromethyl-arylacetic acid of the formula (V) by hydrolysis or cleavage with an acid.

## Revendications

1. Procédé de préparation d'esters d'acides α-alcoxy-α-trifluorométhylarylacétiques de formule (I) 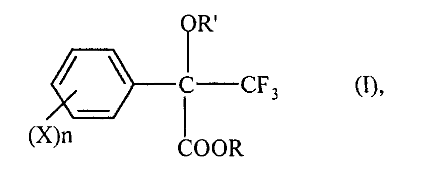 où
R représente alkyle en C₁-C₆, alcényle en C₂-C₆, cycloalkyle en C₃-C₇, aryle en C₆-C₁₀ ou halogénoalkyle en C₁-C₆,
R' représente alkyle en C₁-C₄,
X représente des substituants identiques ou différents du groupe de halogène, alkyle en C₁-C₄, alcényle en C₂-C₄, aryle en C₆-C₁₀ ou alcoxy en C₁-C₄ et nitro
et
n représente 0 ou un nombre entier de 1 à 3,
caractérisé en ce que l'on fait réagir un cétoester de formule (II) 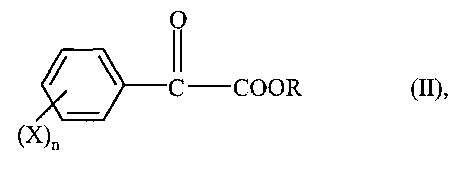 où
R, X et n ont la signification indiquée au sujet de la formule (I),
en présence d'un solvant et d'un fluorure au moins faiblement soluble dans le mélange réactionnel comme catalyseur, avec le trifluorométhyltriméthylsilane, on obtient ainsi un triméthylsilyléther de formule (III) 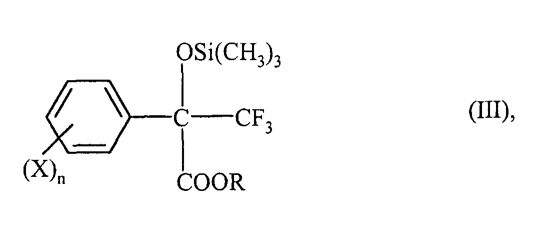 où
R, X et n ont la signification indiquée au sujet de la formule (I),
on fait réagir celui-ci avec un alcoolate ou avec un alcool rendu alcalin, on obtient ainsi un composé hydroxylé de formule (IV) correspondant 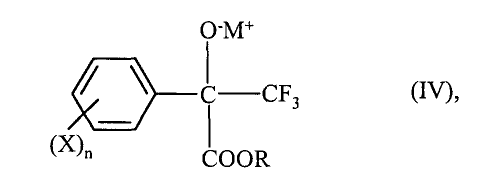 où
R, X et n ont la signification indiquée au sujet de la formule (I) et M⁺ représente l'ion d'un métal alcalin,
et on fait réagir celui-ci avec un agent d'alkylation qui convertit le groupe OH en un groupe OR'.

2. Procédé selon la revendication 1, caractérisé en ce que, dans les formules,
R représente alkyle en C₁-C₄ linéaire ou ramifié, alkylène en C₂-C₄, cyclopentyle, cyclohexyle, phényle ou halogénoalkyle en C₁-C₄ avec 1 à 5 atomes de fluor ou de chlore,
R' représente méthyle, éthyle ou isopropyle,
X représente des substituants identiques ou différents du groupe de méthyle, chlore, brome, éthényle, phényle et nitro,
n représente 0, 1 ou 2.

3. Procédé selon les revendications 1 et 2, caractérisé en ce que le rapport molaire du composé cétonique de formule (II) au trifluorométhyl-triméthylsilane est de 1 : 1 à 1 : 2, on utilise comme solvant pour la réaction du composé cétonique de formule (II) avec le trifluorométhyl-triméthylsilane un solvant aprotique, on utilise comme catalyseur fluorure, 0,05 à 30 mol%, par rapport au cétoester de formule (II), de fluorures alcalins, de bifluorures alcalins, de fluorures d'onium quaternaire, de fluorures chiraux ou de difluorotriméthyl-silicate de tris(diméthylamino)-sulfonium et on conduit la réaction du cétoester de formule (II) avec le trifluorométhyltriméthylsilane à -40 à +120°C.

4. Procédé selon les revendications 1 à 3, caractérisé en ce que l'on utilise comme alcoolates des alcoolates en C₁-C₄ aliphatiques de métaux alcalins ou comme alcools rendus alcalins des alcools en C₁-C₄ aliphatiques qui ont été rendus alcalins avec des hydroxydes alcalins et/ou des carbonates alcalins, on utilise les alcoolates ou les alcools rendus alcalins en une quantité de 1 à 5 équivalents%, par rapport au triméthylsilyléther de formule (III), et on conduit la réaction avec l'alcoolate ou l'alcool rendu alcalin à 0 à 80°C.

5. Procédé selon les revendications 1 à 4, caractérisé en ce que l'on utilise comme agent d'alkylation des agents de méthylation ou des agents d'éthylation connus en soi en une quantité de 1 à 5 équivalents, par rapport au sel d'un composé hydroxylé de formule (IV), et on conduit l'alkylation à des températures de 0 à 100°C.

6. Procédé selon les revendications 1 à 5, caractérisé en ce que, pour l'obtention de l'ester d'acide α-alcoxy-α-trifluorométhyl-arylacétique de formule (I) préparé, on isole le mélange réactionnel présent après l'alkylation par distillation ou par extraction sur l'eau, neutralisation avec un acide, extraction avec un solvant approprié et retrait de l'agent d'extraction.

7. Procédé selon les revendications 1 à 6, caractérisé en ce que l'on purifie encore par distillation l'ester d'acide α-alcoxy-α-trifluorométhyl-arylacétique de formule (I) isolé à partir du mélange réactionnel d'alkylation.

8. Procédé de préparation d'acides α-alcoxy-α-trifluorométhylarylacétiques de formule (V) 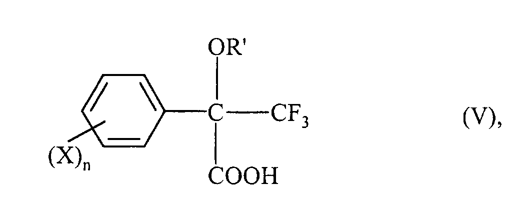 où
R', X et n ont la signification indiquée au sujet de la formule (I) dans la revendication 1, caractérisé en ce que l'on prépare d'abord des esters d'acides α-alcoxy-α-trifluorométhyl-arylacétiques de formule (I) selon les revendications 1 à 8 et on les convertit en un acide α-alcoxy-α-trifluorométhyl-arylacétique de formule (V) par hydrolyse ou clivage avec un acide.
